# EUROPÄISCHE PATENTSCHRIFT

(11) **EP 0 522 242 B1**
(45) Veröffentlichungstag und Bekanntmachung des Hinweises auf die Patenterteilung: **08.11.1995**
(21) Anmeldenummer: 92106490.3
(22) Anmeldetag: 15.04.1992
(51) Int. Cl.: A61B 19/00

(54) **Positioniereinrichtung für ein Körperteil**
Positioning device for a part of the body
Dispositif de positionnement pour une partie du corps

(30) Priorität: 20.06.1991 DE 4120393
(43) Veröffentlichungstag der Anmeldung: 13.01.1993
(73) Patentinhaber: AESCULAP AG, D-78501 Tuttlingen (DE)
(72) Erfinder: Horstmann, Gerhard A., W-7800 Freiburg/Br. (DE); Reinhardt, Hans F., CH-4056 Basel (CH); Weisshaupt, Dieter, W-7717 Immendingen (DE)
(74) Vertreter: Hoeger, Stellrecht & Partner

(56) Entgegenhaltungen:
- DE-B- 2 655 869
- DE-U- 8 802 938
- US-A- 4 465 069
- US-A- 4 838 264

## Beschreibung

Die Erfindung betrifft eine Positioniereinrichtung für ein Körperteil mit mehreren in fester Relativposition zueinander festgelegten Halterungen und mit an jeder Halterung justierbar gehaltenen Fixierelementen zur definierten Anlage an den Körperteil.

Eine Positioniereinrichtung mit den Merkmalen aus dem Oberbegriff des anspruchs 1 ist z.B. bekannt aus DE-B-2 655 869.

Für verschiedene chirurgische Eingriffe ist es notwendig, den zu behandelnden Körperteil in einer exakt definierten Position festzulegen. Dies gilt beispielsweise bei Gehirnoperationen, bei denen der Kopf relativ zu einer Meßeinrichtung exakt positioniert werden muß, die während der Operation die genaue Position der chirurgischen Instrumente bestimmt.

Die genaue Lage bestimmter Körperteile im menschlichen Körper kann heute mit modernen Computeranalysiereinrichtungen, beispielsweise mit Computertomographen, sehr genau bestimmt werden. Es ist daher üblich, vor Operationen die Lage der inneren Körperteile genau zu vermessen. Wenn diese Daten bei der Operation verwendet werden sollen, ist es notwendig, den gesamten Körper in eine genau definierte Position zu bringen, um ausgehend von dieser Position die vorher bestimmten Lagedaten der inneren Körperteile genau verwenden zu können.

13 ist Aufgabe der Erfindung, eine Positionierungeinrichtung für ein Körperteil der gattungsgemäßen Art so auszubilden, daß in ihm eine exakte Positionierung eines beliebigen Körperteiles, beispielsweise des Kopfes, möglich ist und daß diese Positionierung des Körperteils in der Positioniereinrichtung reproduzierbar ist, um beispielsweise bei einer einige Zeit nach einer Computertomographieuntersuchung erfolgenden Operation den Körperteil wieder relativ zur Positioniereinrichtung exakt gleich zu positionieren.

Diese Aufgabe wird bei einer Positioniereinrichtung der eingangs beschriebenen Art erfindungsgemäß dadurch gelöst, daß die Fixierelemente relativ zu den Halterungen in einer gewünschten Position fixierbar gehalten sind und daß die Halterungen ihrerseits lösbar an einem Gestell gelagert sind, an dem sie in einer exakt reproduzierbaren Position befestigbar sind.

In einer solchen Positioniereinrichtung kann ein Körperteil durch Justierung der Fixierelemente in einer genau bestimmbaren Position festgelegt werden. Danach kann die Positioniereinrichtung wieder abgenommen werden, indem die Halterungen vom Gestell gelöst werden. In den Halterungen sind die Fixierelemente nach der Justierung fixiert, so daß beim erneuten Anlegen der Positioniereinrichtung nur die Halterungen wieder reproduzierbar am Gestell festgelegt werden müssen, um eine genau reproduzierbare Positionierung des Körperteils in der Einrichtung zu gewährleisten. Die eigentliche Justierung erfolgt also zwischen Fixierelement und Halterung, und diese Fixierung bleibt auch bei Abnahme der Positioniereinrichtung erhalten.

Besonders vorteilhaft ist es, wenn die Fixierelemente Stäbe sind, die längsverschieblich in den als Hülsen ausgebildeten Halterungen gelagert sind. Zur Justierung können die stabförmigen Fixierelemente in den Hülsen längsverschoben werden, in der gleichen Richtung lassen sich dann die hülsenförmigen Halterungen mitsamt den justierten stabförmigen Fixierelementen aus dem Gestell entnehmen, so daß beispielsweise beim Eingreifen der stabförmigen Fixierelemente in Bohrungen des Körperteils ohne weiteres eine Abnahme der Positioniereinrichtung möglich ist.

Bei dem bevorzugten Ausführungsbeispiel ist vorgesehen, daß die stabförmigen Fixierelemente durch eine Spanneinrichtung in der Hülse gegen Längsverschiebung fixierbar sind. Dabei ist es vorteilhaft, wenn die Spanneinrichtung an der Hülse angeformte, elastisch an das stabförmige Fixierelement angelegte Klemmbacken umfaßt, die durch eine Überwurfmutter elastisch an das Fixierelement andrückbar sind. Insbesondere kann die Überwurfmutter auf ein Außengewinde der Hülse aufgeschraubt sein.

Günstig ist es dabei, wenn die Klemmbacken mit Innengewindegängen versehen sind, die zu einem Außengewinde des Fixierelementes passen. Beim Klemmen der Klemmbacken erfolgt somit nicht nur ein Kraftschluß, sondern ein Kraftformschluß, der die Fixierelemente mit höchstmöglicher Sicherheit gegen eine unbeabsichtigte Verschiebung sichert. Außerdem ist es möglich, bei nicht vollständig gespannter Spanneinrichtung zur Justierung die stabförmigen Fixierelemente durch Verschrauben in axialer Richtung zu verschieben, d.h. auf diese Weise kann eine Feinjustierung erfolgen. Bei vollständig gelöster Spannzange hingegen greifen die Klemmbacken überhaupt nicht in das Außengewinde des stabförmigen Fixierelementes ein, dann kann zur Grobjustierung das Fixierelement in der Hülse in axialer Richtung frei verschoben werden.

Das Fixierelement kann an seinem körperseitigen Ende einen Fixierstift tragen, der beispielsweise in eine Sacklochbohrung in einem Knochen des zu positionierenden Körperteils eintauchen kann. Um derartige Sacklochbohrungen herzustellen, kann auch vorgesehen sein, daß Fixierelemente eine Durchgangsbohrung aufweisen. Diese Fixierelemente können dann als Bohrlehren für die gewünschten Sacklochbohrungen verwendet werden, nach dem Bohrvorgang werden diese Fixierelemente durch Fixierelemente mit einem Fixierstift ersetzt, der dann in die entsprechende Sacklochbohrung eintaucht.

Die Hülse wird vorzugsweise in eine die Hülse umschließende Öffnung des Gestells axial eingesetzt und in dieser gegen axiale Verschiebung gesichert.

Dies kann bei einer bevorzugten Ausführung z.B. dadurch erfolgen, daß die Hülse einerseits einen radial vorstehenden Anschlag und andererseits eine Umfangsnut aufweist, in die nach dem Einsetzen der Hülse in das Gestell ein Anschlag seitlich einschiebbar ist, und daß der Abstand der beiden Anschläge exakt der Dicke des Gestells im Bereich der die Hülse aufnehmenden Öffnung entspricht. Allein durch Einschieben des Anschlags in die Umfangsnut erfolgt somit eine axiale Festlegung, umgekehrt kann nach Entfernung des in die Umfangsnut eingeschobenen Anschlags die Hülse leicht aus dem Gestell entnommen werden.

Der in die Umfangsnut einschiebbare Anschlag kann vorzugsweise ein U-förmiger Bügel sein, dessen beide Arme einen Abstand voneinander haben, der dem Durchmesser des Fixierelements im Bereich der Umfangsnut entspricht.

Bei dem bevorzugten Ausführungsbeispiel ist vorgesehen, daß das Gestell für jede Halterung einen Pfosten umfaßt und daß alle Pfosten an einem Rahmen befestigt sind. Dabei können die Pfosten am Rahmen in verschiedenen Positionen befestigbar sein, so daß je nach der Form des zu positionierenden Körperteils die Pfosten in eine besonders günstige Position gebracht werden können.

Zusätzlich kann der Rahmen mindestens eine Anlagefläche für das zu positionierende Körperteil tragen, so daß bei Beginn des Positioniervorgangs bereits eine Rohpositionierung des Körperteils relativ zum Gestell erfolgen kann, die dann durch Justierung der Fixierelemente verfeinert wird.

Die nachfolgende Beschreibung bevorzugter Ausführungsformen der Erfindung dient im Zusammenhang mit der Zeichnung der näheren Erläuterung. Es zeigen:
- Fig. 1: eine Draufsicht auf eine Positioniereinrichtung mit drei je einen Fixierstift tragenden Fixierelementen und mit einem als Bohrlehre ausgebildeten Fixierelement;
- Fig. 2: eine Schnittansicht längs Linie 2-2 in Fig. 1 und

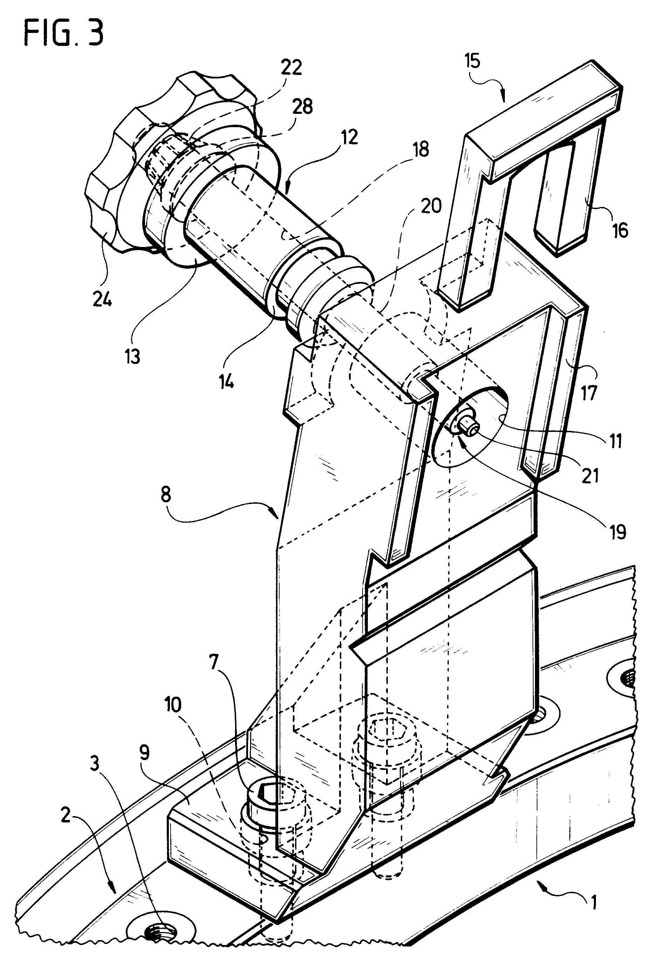

- Fig. 3: eine perspektivische Ansicht eines Pfostens der Positioniereinrichtung mit einer herausgenommenen Halterung für ein Fixierelement.

Die in der Zeichnung dargestellte Positioniereinrichtung 1 umfaßt einen kreisringförmigen Rahmen 2, längs dessen Umfang eine größere Anzahl von Schraubgewindelöchern 3 äquidistant verteilt sind.

In einige dieser Schraubgewindelöcher 3 ist an einer Seite des Rahmens 2 ein Stützpfosten 4 mittels Schrauben festgelegt, der eine Anlagefläche 5 für ein Körperteil 6 trägt. Das Körperteil 6 kann bespielsweise der Kopf eines Menschen sein, in der Zeichnung ist der Körperteil 6 lediglich durch eine strichpunktierte Umfangslinie symbolisiert.

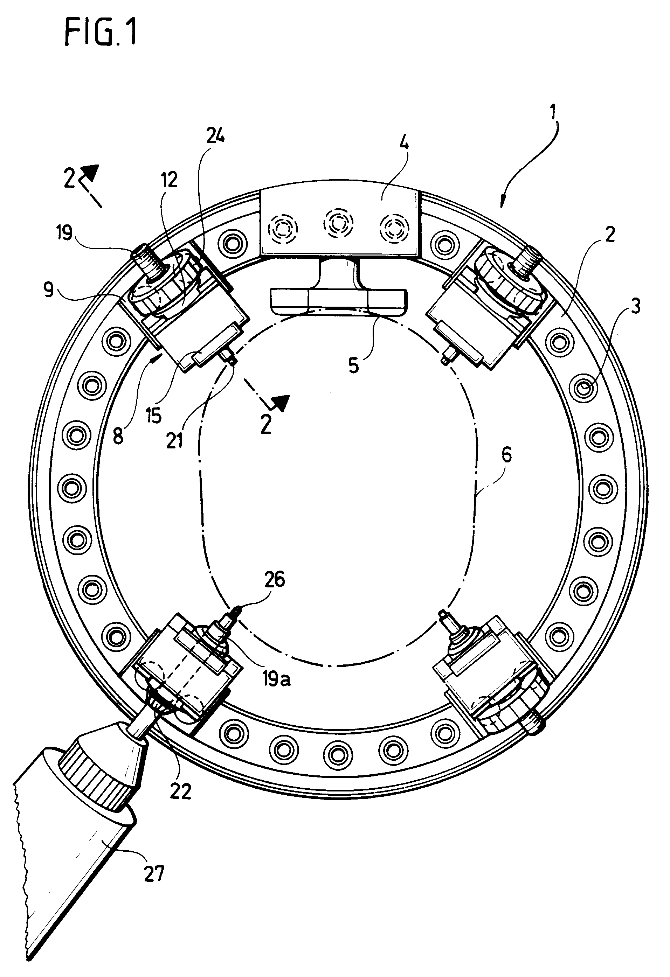

Mittels je zwei Befestigungsschrauben 7 sind an dem Rahmen 2 weiterhin vier Pfosten 8 so befestigt, daß sie den Körperteil 6 umgeben, wie dies aus Fig. 1 deutlich wird. Jeder dieser Pfosten 8 weist einen Fuß 9 mit einer Durchgangsbohrung 10 für die Befestigungsschraube 7 auf, sowie an seinem oberen Ende, in dem der Pfosten geringfügig gegenüber der Senkrechten nach innen geneigt ist, eine Durchgangsöffnung 11, die den Pfosten senkrecht von außen nach innen durchsetzt.

In diese Durchgangsöffnung 11 ist eine hülsenförmige Halterung 12 eingeschoben, deren Außendurchmesser dem Innendurchmesser der Durchgangsöffnung 11 entspricht, so daß die Halterung 12 flächig an der Innenwand der Durchgangsöffnung 11 anliegt. Die Einschubtiefe der Halterung 12 wird durch einen an der Außenseite des Pfostens 8 anliegenden Flansch 13 begrenzt, auf der Innenseite ragt die Halterung 12 bei der maximalen Einschubtiefe aus der Durchgangsöffnung 11 hervor und trägt in dem hervorstehenden Bereich eine Umfangsnut 14, in die seitlich ein U-förmiger Bügel 15 eingeschoben ist, dessen Arme 16 einen Abstand voneinander haben, der dem Außendurchmesser der Halterung 12 im Bereich der Umfangsnut 14 entspricht. Der Abstand der Umfangsnut 14 einerseits und des Flansches 13 andererseits ist so gewählt, daß die hülsenförmige Halterung bei eingeschobenem Bügel 15 in der Durchgangsöffnung 11 in axialer Richtung spielfrei festgelegt ist.

Der Pfosten 8 ist im Bereich der Durchgangsöffnung 11 auf der Innenseite leistenförmig vorgezogen, so daß die dadurch entstehenden Vorsprünge 17 sich außenseitig an die Arme 16 des eingeschobenen Bügels 15 anlegen, so daß der Bügel 15 eine Führung erfährt und in der eingeschobenen Stellung gegen Drehung um die Längsachse der Durchgangsöffnung 11 fixiert ist.

In eine zylindrische Durchstecköffnung 18 der hülsenförmigen Halterung 12 ist ein stabförmiges Fixierelement 19 eingeschoben, welches ein Außengewinde 20 trägt, dessen Außendurchmesser genau dem Innendurchmesser der Durchstecköffnung 12 entspricht. Das Fixierelement 19 ist auf der dem Körperteil 6 zugewandten Seite stufenförmig verjüngt und trägt an seinem freien Ende einen Fixierstift 21.

An den Flansch 13 der Halterung 12 und eine sich daran anschließende Verlängerung 28 mit Außengewinde 29 sind nach außen abstehende Klemmbacken 22 angeformt, die an dem eingeschobenen Fixierelement 19 anliegen und in radialer Richtung elastisch verschiebbar sind. Die Klemmbacken 22 tragen an ihrer dem Fixierelement 19 zugewandten Seite Gewindegänge 23, die dem Außengewinde 20 des Fixierelementes 19 angepaßt sind, an der Außenseite sind die Klemmbacken 22 konisch ausgebildet, d.h. sie verjüngen sich nach außen hin nach Art eines Kegelstumpfes.

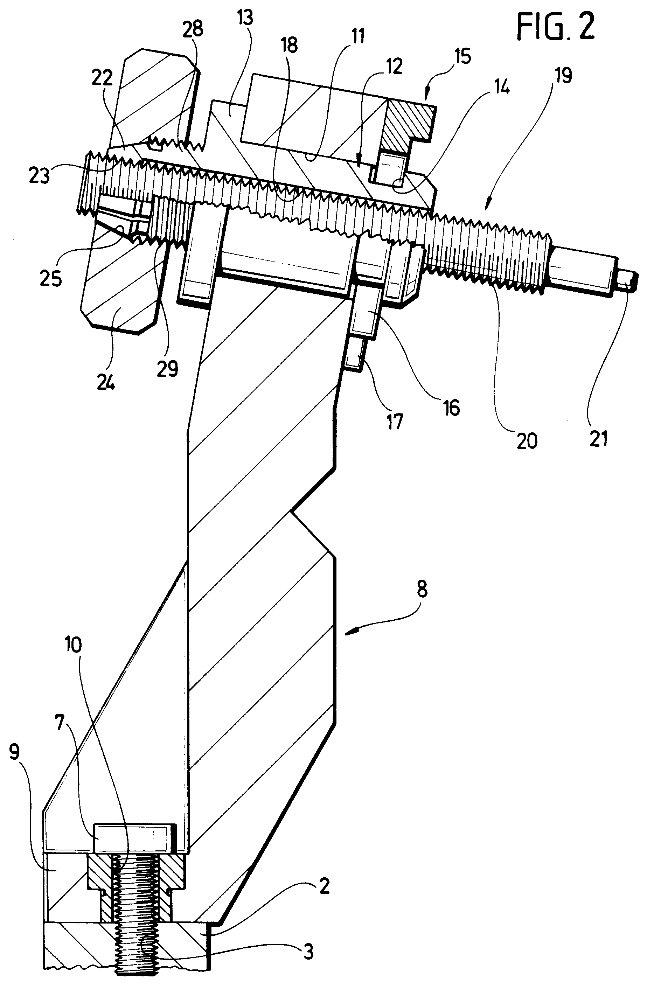

Auf das Außengewinde 29 der Verlängerung 28 ist ein Handrad 24 aufgeschraubt, welches eine konische, das Fixierelement 19 konzentrisch umgebende Spannfläche 25 trägt, die beim Aufschrauben auf das Außengewinde 29 an der Außenseite der Klemmbacken 22 zur Anlage kommt und dadurch die Klemmbacken 22 radial nach innen spannt, wie dies aus der Darstellung der Fig. 2 ersichtlich ist.

Das bisher beschriebene Fixierelement 19 ist in der Regel massiv ausgebildet und trägt einen Fixierstift 21.

Es kann ersetzt werden durch ein stabförmiges Bohrlehren-Fixierelement 19a, das keinen Fixierstift 21 trägt und dafür eine durchgehende Längsbohrung aufweist, durch die der Bohrer 26 einer Bohrmaschine 27 hindurchgesteckt werden kann (Fig. 1).

Die Fixierelemente 19 und 19a - sowohl in der Ausführung mit Fixierstift 21 als auch in der Ausführung als Bohrlehre - sowie die hülsenförmige Halterung mit aufgeschraubtem Handrand 24 werden vorzugsweise aus einem sterilisierbaren Kunststoffmaterial hergestellt.

Zur Verwendung der beschriebenen Positioniereinrichtung wird diese zunächst mit einer geeigneten, in der Zeichnung nicht dargestellten zusätzlichen Halterung, beispielsweise mittels eines verschwenkbaren Armes, in eine Position gebracht, in welcher der Rahmen 2 den zu positionierenden Körperteil 6 umgibt, dazu wird der Körperteil 6 an die Anlagefläche 5 angelegt. Anschließend werden in die Durchgangsöffnungen 11 der bereits vorher auf den Rahmen 2 aufgeschraubten Pfosten 8 Halterungen 12 eingesetzt, in die Bohrlehren-Fixierelemente eingesteckt sind. Diese werden so weit vorgeschoben, bis sie mit ihrem vorderen Ende an dem zu positionierenden Körperteil anliegen. Mittels der Bohrmaschine 27 werden daraufhin mit Hilfe dieser Bohrlehren Sacklochbohrungen in den Körperteil eingebracht, und zwar mit definierter Tiefe.

Nach Abschluß des Bohrvorganges werden die Bohrlehren-Fixierelemente ersetzt durch Fixierelemente 19 mit Fixierstift 21, und diese Fixierelemente 19 werden so in den hülsenförmigen Halterungen 12 verschoben, daß die Fixierstifte 21 in die vorher gebohrten Sacklochbohrungen im Körperteil 6 eintauchen. Dabei entspricht der Außendurchmesser des Fixierstifts 21 dem Innendurchmesser dieser Sacklochbohrungen.

Auf diese Weise wird der Körperteil durch die Fixierelemente 19 gemeinsam exakt positioniert. Die Justierung der Fixierelemente erfolgt dabei zunächst bei zurückgeschraubtem Handrad 24, so daß die Klemmbacken 22 vom Fixierelement 19 abgebogen sind. Das Fixierelement 19 läßt sich dadurch in axialer Richtung frei in der Durchgangsöffnung der Halterung 12 verschieben. Sobald die Fixierelemente grob positioniert sind, wird das Handrad 24 etwas aufgeschraubt, wodurch die Klemmbacken 22 nach innen gebogen werden, so daß sich ein Formschluß zwischen dem Außengewinde 20 des Fixierelementes 19 und den Gewindegängen 23 der Klemmbacken 22 ergibt. Das Fixierelement 19 kann dadurch in der durch die Klemmbacken gebildeten Spannzange verschraubt werden, es ist somit eine Axialfeinverschiebung möglich. Sobald die endgültige Position erreicht ist, wird das Handrad 24 fest aufgedreht, dadurch ergibt sich für das Fixierelement 19 zusätzlich eine Klemmung, es ist gegenüber der Halterung dann durch einen Kraft-Form-Schluß unverschiebbar festgelegt.

Nach dieser Positionierung des Körperteils relativ zu der Positioniereinrichtung 1 kann diese in einfacher Weise vom Körperteil wieder dadurch abgenommen werden, daß an jedem Pfosten die Bügel 15 aus den entsprechenden Umfangsnuten 14 herausgezogen werden. Sobald dies erfolgt ist, lassen sich die hülsenförmigen Halterungen 12 axial aus den Durchgangsöffnungen 11 der Pfosten 8 herausnehmen, wobei die Halterungen 12 sowie die unverschiebbar darin festgelegten Fixierelemente 19 eine Einheit bilden.

Die Positioniereinrichtung 1 läßt sich in umgekehrter Weise wieder am Körperteil anlegen, indem die in der beschriebenen Weise vorjustierten Baueinheiten aus Halterung 12 und Fixierelement 19 wieder in die Durchgangsöffnungen 11 der Pfosten 8 eingeschoben werden. Um hier eine einwandfreie Zuordnung zu gewährleisten, können beispielsweise Farbmarkierungen an den Halterungen und den zugehörigen Pfosten angeordnet sein, bei anderen Ausführungsformen können die Geometrie der Durchgangsöffnung 11 und der Halterung 12 so aneinander angepaßt sein, daß eine bestimmte Halterung nur bei einem bestimmten Pfosten eingesetzt werden kann. Dies läßt sich beispielsweise durch unterschiedliche Durchmesser der Halterungen oder durch unterschiedliche Querschnittsformen der Halterung und der zugehörigen Durchgangsöffnungen erreichen, beispielsweise können die Durchgangsöffnungen quadratisch, dreieckig, sechseckig etc. ausgebildet sein.

Sobald die vorjustierten Baueinheiten aus Halterung und Fixierelement in den entsprechenden Pfosten eingesetzt sind, werden sie in diesen durch Einschieben des Bügels 15 fixiert, und dadurch wird der Körperteil, in dessen Sacklochbohrungen die Fixierstifte 21 der Fixierelemente 19 eingreifen, reproduzierbar in derselben Position relativ zu der Positioniereinrichtung 1 festgelegt, die bei der Justierung der Fixierelemente 19 in den Halterungen 12 eingenommen worden war.

Es ist ein besonderer Vorteil der beschriebenen Positioniereinrichtung, daß die Daten für die einmal vorgenommene Positionierungsjustierung praktisch in den Baueinheiten aus Halterung und Fixierelement gespeichert werden, die von der sehr sperrigen Positioniereinrichtung getrennt aufbewahrt werden können und somit individuell für jeden Patienten einen körperlichen Datensatz für die Positionierung des Körperteils in der Positioniereinrichtung bilden. Dieser Datensatz kann für jeden Patienten getrennt aufbewahrt und mit einer allgemein verwendbaren Positioniereinrichtung eingesetzt werden. Dies führt zu einer wesentlichen Einsparung an Material und damit zu einer deutlichen Kostensenkung, außerdem wird beispielsweise die Sterilisation der mit dem Körperteil in Berührung kommenden Teile wesentlich vereinfacht, da sich diese auf die patientenspezifischen Baueinheiten aus Halterung und Fixierelementen beschränken kann.

## Patentansprüche

1. Positioniereinrichtung für ein Körperteil mit mehreren in fester Relativposition zueinander festgelegten Halterungen (12) und mit an jeder Halterung justierbar gehaltenen Fixierelementen (19, 19A) zur definierten Anlage an den Körperteil,
dadurch gekennzeichnet, daß die Fixierelemente (19; 19a) relativ zu den Halterungen (12) in einer gewünschten Position fixierbar gehalten sind und daß die Halterungen (12) ihrerseits lösbar an einem Gestell (2) gelagert sind, an dem sie in einer exakt reproduzierbaren Position befestigbar sind.

2. Einrichtung nach Anspruch 1, dadurch gekennzeichnet, daß die Fixierelemente (19; 19a) Stäbe sind, die längsverschieblich in den als Hülsen ausgebildeten Halterungen (12) gelagert sind.

3. Einrichtung nach Anspruch 2, dadurch gekennzeichnet, daß die stabförmigen Fixierelemente (19) durch eine Spanneinrichtung (22, 24) in der hülsenförmigen Halterung (12) gegen Längsverschiebung fixierbar sind.

4. Einrichtung nach Anspruch 3, dadurch gekennzeichnet, daß die Spanneinrichtung an der hülsenförmigen Halterung (12) angeformte, elastisch an das stabförmige Fixierelement (19) angelegte Klemmbacken (22) umfaßt, die durch eine Überwurfmutter (24) elastisch an das Fixierelement (19) andrückbar sind.

5. Einrichtung nach Anspruch 4, dadurch gekennzeichnet, daß die Überwurfmutter (24) auf ein Außengewinde (29) der hülsenförmigen Halterung (12) aufgeschraubt ist.

6. Einrichtung nach Anspruch 5, dadurch gekennzeichnet, daß die Klemmbacken (22) mit Innengewindegängen (23) versehen sind, die zu einem Außengewinde (20) des Fixier- elementes (19) passen.

7. Einrichtung nach einem der voranstehenden Ansprüche, dadurch gekennzeichnet, daß das Fixierelement (19) an seinem körperseitigen Ende einen Fixierstift (21) trägt.

8. Einrichtung nach einem der Ansprüche 1 oder 2, dadurch gekennzeichnet, daß das Fixierelement (19a) eine Durchgangsbohrung aufweist.

9. Einrichtung nach einem der Ansprüche 2 bis 8, dadurch gekennzeichnet, daß die hülsenförmige Halterung (12) in eine die Halterung umschließende Öffnung (11) des Gestells (8) axial eingesetzt und in dieser gegen axiale Verschiebung gesichert ist.

10. Einrichtung nach Anspruch 9, dadurch gekennzeichnet, daß die hülsenförmige Halterung (12) einerseits einen radial vorstehenden Anschlag (13) und andererseits eine Umfangsnut (14) aufweist, in die nach dem Einschieben der Halterung (12) in das Gestell (8) ein Anschlag (15) seitlich einschiebbar ist, und daß der Abstand der beiden Anschläge (13, 15) der Dicke des Gestells (8) im Bereich der die Halterung (12) aufnehmenden Öffnung (11) entspricht.

11. Einrichtung nach Anspruch 10, dadurch gekennzeichnet, daß der in die Umfangsnut (14) einschiebbare Anschlag (15) ein U-förmiger Bügel ist, dessen beide Arme (16) einen Abstand voneinander haben, der dem Durchmesser des Fixierelementes (19) im Bereich der Umfangsnut (14) entspricht.

12. Einrichtung nach einem der voranstehenden Ansprüche, dadurch gekennzeichnet, daß das Gestell für jede Halterung (12) einen Pfosten (8) umfaßt und daß alle Pfosten (8) an einem Rahmen (2) befestigt sind.

13. Einrichtung nach Anspruch 12, dadurch gekennzeichnet, daß die Pfosten (8) am Rahmen (2) in verschiedenen Positionen befestigbar sind.

14. Einrichtung nach einem der Ansprüche 12 oder 13, dadurch gekennzeichnet, daß der Rahmen (2) mindestens eine Anlagefläche (5) für das zu positionierende Körperteil (6) trägt.

## Claims

1. A positioning device for a part of the body, comprising a plurality of mountings (12) secured in a fixed position relative to each other and comprising locating members (19, 19a) adjustably held on each mounting for defined location on the body part, characterised in that the locating members (19, 19a) are securably held in a desired position relative to the mountings (12) and in that the mountings (12) are detachably mounted on a framework (2) and are fixable thereto in an exactly reproducible position.

2. A device according to claim 1, characterised in that the locating members (19, 19a) are rods mounted so as to be longitudinally displaceable in the mountings (12) formed as sleeves.

3. A device according to claim 2, characterised in that the rod-shaped locating members (19) are securable against longitudinal displacement in the sleeve-type mounting (12) by a clamping device (22, 24).

4. A device according to claim 3, characterised in that the clamping device comprises clamping jaws (22) formed on the sleeve-type mounting (12) and lying resiliently against the rod-shaped locating member (19), the said clamping jaws (22) being resiliently pressable against the locating member (19) by a sleeve nut (24).

5. A device according to claim 4, characterised in that the sleeve nut (24) is screwed onto an external thread (29) of the sleeve-type mounting (12).

6. A device according to claim 5, characterised in that the clamping jaws (22) are provided with internal threads (23) matching an external thread (20) of the locating member (19).

7. A device according to any one of the preceding claims, characterised in that the locating member (19) carries a locating pin (21) at its end near the body.

8. A device according to either one of claims 1 or 2, characterised in that the locating member (19a) has a through-hole.

9. A device according to any one of claims 2 to 8, characterised in that the sleeve-type mounting (12) is inserted axially into an opening (11) in the framework (8) and is secured therein against axial displacement, the said opening (11) surrounding the said mounting.

10. A device according to claim 9, characterised in that the sleeve-type mounting (12) has a radially projecting stop (13) at one end and, at the other end, a circumferential groove (14) in which a stop (15) is laterally engageable after the mounting (12) has been inserted into the framework (8), and in that the distance between the two stops (13, 15) corresponds to the thickness of the framework (8) in the region of the opening (11) receiving the mounting (12).

11. A device according to claim 10, characterised in that the stop (15), engageable in the circumferential groove (14), is a U-shaped stirrup, the two arms (16) thereof being at a distance from each other which corresponds to the diameter of the locating member (19) in the region of the circumferential groove (14).

12. A device according to any one of the preceding claims, characterised in that the framework comprises a support (8) for each mounting (12) and in that all the supports (8) are fixed to a frame (2).

13. A device according to claim 12, characterised in that the supports (8) are fixable to the frame (2) in different positions.

14. A device according to either one of claims 12 or 13, characterised in that the frame (2) has at least one supporting surface (5) for the body part (6) to be positioned.

## Revendications

1. Dispositif de positionnement pour une partie du corps, comportant plusieurs montures (12) fixées les unes par rapport aux autres dans une position relative fixe, et des éléments de fixation (19 ; 19a) retenus de façon ajustable sur chaque monture pour l'appui défini contre la partie du corps,
caractérisé en ce que les éléments de fixation (19 ; 19a) sont retenus en pouvant être fixés dans une position désirée par rapport aux montures (12), et en ce que les montures (12) sont montées à leur tour de façon détachable sur un châssis (2), sur lequel elles peuvent être fixées dans une position exactement reproductible.

2. Dispositif selon la revendication 1, caractérisé en ce que les éléments de fixation (19 ; 19a) sont des barres qui sont montées en déplacement longitudinal dans les montures (12) réalisées sous la forme de douilles.

3. Dispositif selon la revendication 2, caractérisé en ce que les éléments de fixation (19) en forme de barres peuvent être fixés par un dispositif de serrage (22, 24) dans la monture (12) en forme de douille à l'encontre l'un déplacement longitudinal.

4. Dispositif selon la revendication 3, caractérisé en ce que le dispositif de serrage comprend des mâchoires de serrage (22) formées sur la monture (12) en forme de douille et appliquées élastiquement contre l'élément de fixation (19) en forme de barre, qui peuvent être appuyées élastiquement contre l'élément de fixation (19) par un écrou-raccord (24).

5. Dispositif selon la revendication 4, caractérisé en ce que l'écrou-raccord (24) est vissé sur un filetage (29) de la monture (12) en forme de douille.

6. Dispositif selon la revendication 5, caractérisé en ce que les mâchoires de serrage (22) sont pourvues de taraudages (23) qui s'ajustent sur un filetage (20) des éléments de fixation (19).

7. Dispositif selon l'une quelconque des revendications précédentes, caractérisé en ce que l'élément de fixation (19) porte à son extrémité côté corps une tige de fixation (21).

8. Dispositif selon l'une ou l'autre des revendications 1 et 2, caractérisé en ce que l'élément de fixation (19a) présente un perçage traversant.

9. Dispositif selon l'une quelconque des revendications 2 à 8, caractérisé en ce que la monture (12) en forme de douille est axialement mise en place dans une ouverture (11) du châssis (8), qui entoure la monture, et en ce qu'elle est bloquée dans celle-ci à l'encontre d'un déplacement axial.

10. Dispositif selon la revendication 9, caractérisé en ce que la monture (12) en forme de douille présente d'une part une butée (13) faisant radialement saillie, et d'autre part une gorge périphérique (14) dans laquelle on peut introduire latéralement une butée (15), après l'introduction de la monture (12) dans le châssis (8), et en ce que la distance des deux butées (13, 15) correspond à l'épaisseur du châssis (8) dans la région de l'ouverture (11) qui reçoit la monture (12).

11. Dispositif selon la revendication 10, caractérisé en ce que la butée (15) qui peut être introduite dans la gorge périphérique (14) est une anse en forme de U, dont les deux bras (16) présentent l'un par rapport à l'autre une distance qui correspond au diamètre de l'élément de fixation (19) dans la région de la gorge périphérique (14).

12. Dispositif selon l'une quelconque des revendications précédentes, caractérisé en ce que le châssis comprend pour chaque monture (12) un montant (8), et en ce que tous les montants (8) sont fixés sur un cadre (2).

13. Dispositif selon la revendication 12, caractérisé en ce que les montants (8) peuvent être fixés sur le cadre (2) dans différentes positions.

14. Dispositif selon l'une ou l'autre des revendications 12 et 13, caractérisé en ce que le cadre (2) porte au moins une surface d'appui (5) pour la partie du corps (6) à positionner.
